# EUROPEAN PATENT SPECIFICATION

(11) **EP 2 501 438 B1**
(45) Date of publication and mention of the grant of the patent: **28.03.2018**
(21) Application number: 10776721.2
(22) Date of filing: 15.11.2010
(51) Int. Cl.: A61Q 17/04, A61K 8/02, A61K 8/04, A61K 8/29, A61K 8/37, A61K 8/39

(54) **TOPICAL COMPOSITIONS**
TOPISCHE ZUSAMMENSETZUNGEN
COMPOSITIONS TOPIQUES

(30) Priority: 18.11.2009 EP 09176310
(43) Date of publication of application: 26.09.2012
(73) Proprietor: DSM IP Assets B.V., 6411 TE Heerlen (NL)
(72) Inventor: SATZINGER, Thomas, CH-4002 Basel (CH); WESTENFELDER, Horst, CH-4002 Basel (CH)
(74) Representative: Berg, Katja
(86) International application number: PCT/EP2010/067433
(87) International publication number: WO 2011/061133

(56) References cited:
- EP-A1- 1 579 847
- WO-A1-2006/087066
- WO-A2-2007/065574
- DE-A1- 19 735 055
- DE-A1-102007 005 093
- DE-A1-102007 005 335
- DE-A1-102007 024 343

## Description

The present invention relates to a method of improvement of the water resistance of micronized double coated titanium dioxide particles having an inner inorganic silica coating and an outer silicone coating in a topical composition characterized in that said particles are incorporated into the topical composition in the form of a dispersion of said particles in C₁₂₋₁₅ alkyl benzoate and polyglyceryl-2 dipolyhydroxystearate. Furthermore, the invention relates to the topical compositions comprising said dispersions as well as to the use thereof as sunscreen.

There is a constantly increasing need for sunscreens comprising constantly increasing amounts of UV-filter substances in order to provide effective protection against UV-radiation. Such sunscreens should exhibit a high SPF while exhibiting a sufficient water resistance and an appealing skin feel.

Inorganic micronized titanium dioxide UV-filters are particularly useful in sunscreen applications due to their ability to increase the sun protection factor (SPF) of formulations over a broad UV range from 250 to 380 nm. In addition, micronized titanium dioxides are generally regarded as safe for cosmetic use, and do not have the disadvantage of a tacky skin-feel as is the case with most organic UV filters. Nevertheless, there are still many problems associated with the use of micronized titanium dioxide UV-filters. In particular, the level of titanium dioxide powder necessary to achieve proper (and higher) SPF levels makes the product aesthetically unacceptable, i.e. there is a heavy and gritty feel and a white/blue residual on the skin. Furthermore, the wash off resistance of inorganic micronized titanium dioxide particles in topical compositions is not yet satisfying in view of the formulation of 'very water resistant' (also labeled as waterproof) sunscreens.

Surprisingly, it has been found that topical compositions comprising an organic titanium dioxide dispersion said dispersion consisting of double coated micronized titanium dioxide particles having one inner inorganic silica coating and one outer silicone coating dispersed in a mixture of C₁₂₋₁₅ alkyl benzoate (CAS-No.: 68411-27-8) and polyglyceryl-2 dipolyhydroxystearate (CAS-No.: 137398-08-4) overcome the drawbacks of the prior art. In particular, topical compositions comprising said dispersion exhibit improved sun protection factors (SPF) and improved water-resistance. As an additional benefit, the topical compositions comprising said organic titanium dioxide dispersions exhibit an excellent skin feel, show an enhanced transparency thus avoiding the so called 'whitening effect' on the skin and do not show a discoloration when used in combination with dibenzoylmethane derivatives.

Thus, in one embodiment the invention relates to topical compositions comprising in a cosmetically acceptable carrier an organic titanium dioxide dispersion, characterized in that said dispersion consists of double coated micronized titanium dioxide particles having one inner inorganic silica coating and one outer silicone coating (in the following referred to as double coated titanium dioxide particles) dispersed in a mixture of C₁₂₋₁₅ alkyl benzoate and polyglyceryl-2 dipolyhydroxystearate.

The term 'consists of' as used according to the present invention refers to dispersions wherein the total amount of the ingredients sum up to 100 wt.-%. However, the term 'consists of' does not exclude trace amounts of additional additives or impurities which are e.g. introduced via the respective raw materials which, however, should not exceed 5 wt.-%, in particular 3 wt.-% based on the total weight of the dispersion.

In a particular embodiment, the organic titanium dioxide dispersion used according to the present invention consists of 10-80 wt.-% of double coated titanium dioxide particles, 10-80 wt.-% of C₁₂₋₁₅ alkyl benzoate and 0.5-20 wt.-% of polyglyceryl-2 dipolyhydroxystearate. Particularly, the organic titanium dioxide dispersion consists of 25-60 wt.-% of double coated titanium dioxide particles, 30-70 wt.-% of C₁₂₋₁₅ alkyl benzoate and 2-15 wt.-% of polyglyceryl-2 dipolyhydroxystearate. Most in particular, the organic titanium dioxide dispersion consists of 45-55 wt.-% of double coated titanium dioxide particles, 40-50 wt.-% of C₁₂₋₁₅ alkyl benzoate and 6-8 wt.-% of polyglyceryl-2 dipolyhydroxystearate. Most in particular the content of the double coated titanium dioxide particles in the dispersion is about 50 wt.-%.

All percentages and ratios mentioned in this specification are by weight if nothing else is stated or evident.
It has furthermore been found, that the inventive advantages are in particular pronounced when the ratio of double coated titanium dioxide particles to polyglyceryl-2 dipolyhydroxystearate in the dispersion is selected in the range of 20 to 1 to 10 to 1, most in particular 10 to 1 to 5 to 1.
The double coated titanium dioxide particles according to the invention can be prepared according to the process described in example 1 of EP 444798.
The inner coating of the titanium dioxide with inorganic silica can be prepared according to the state of the art as e.g. described in EP 44515, EP-A 988 853, EP-A 1 284 277, EP0988853, and US 5562897, JP 2000319128 as well as according to DE10333029. The inner inorganic silica coating layer is present in an amount of at least 0.5 wt %, preferably in an amount of 0.5-50 wt.-%, most preferably in an amount of 1-20 wt.-% based on uncoated titanium dioxide.
The outer silicone coating is selected from the class of known silicone coatings such as e.g. silane derivatives such as triethoxycaprylylsilane or siloxane derivatives such as silicone oils. In particular suitable silicone oils are polydimethylsiloxanes (also known as dimethicone such as e.g. the polydimethylsiloxanes with the CAS No. [CAS 63148-62-9] or [CAS 9016-00-6], simethicones [CAS 8050-81-5], polymethylhydrosiloxanes (also known as methicones [CAS 9004-73-3]) and polysilicone-15. In particular the outer coating is selected from polydimethylsiloxanes [CAS 63148-62-9] or [CAS 9016-00-6]. The outer coating layer consists of minimum 0.25 wt. % based on the titanium dioxide. Preferably the outer coating layer consists of 0.5-50 wt. %, most preferably of 0.5-10 wt. % based on titanium dioxide.
The surface of the titanium dioxide can be pretreated before the coating in order to additionally reduce the surface activity. Such pretreatments are well known to a person skilled in the art and can be performed e.g. with (a) fluoro acids selected from H₂SiF₆, H₂TiF₆, H₂ZrF₆, H₂HfF₆, H₂GeF₆, H₂SnF₆, and/or HBF₄; (b) water-sol. carboxylic acid contg. ≥ 2 hydroxyl groups per carboxyl group in each acid mol.(esp. gluconic acid); (c) water-sol. salts of such carboxylic acids; (d) source of phosphate ions, esp. H₃PO₂ and/or phosphate salts and/ or organophosphoric acids and their salts; (e) inorg. acid such as H₂SO₂, HNO₃, H₃PO₂, hydrobromic, hydroiodic and or perchloric acid (f) org. component selected from tannins and/or amino-phenolic polymers; and (h) optional oxide, hydroxide.
Preferably no pre-treatment or a pre-treatment with a source of phosphate ions, esp. H₃PO₄ and/or phosphate salts and/ or organophosphoric acids and their salts is applied.
The term "double coated" as used according to the invention denotes to the presence of two coatings on the titanium dioxide particles, i.e., an inner inorganic silica coating and an outer silicone coating. Preferably, the outer coating consists of methicone, polydimethylsiloxane or mixtures thereof; in particular the outer coating is polydimethylsiloxane.
Most preferably, the double coated titanium dioxide particles are in the form of a white powder consisting of titanium oxide of rutile crystal structure coated with silica and polydimethylsiloxane (CAS 63148-62-9) which is commercially available under the tradename PARSOL® TX by DSM Nutritional Products Ltd.
The term micronized titanium dioxide particles refers to titanium dioxide particles having a particle sizes which are principally useful for incorporation into sunscreen compositions as UV-filters. Such particles preferably have a primary particle size in the range from 2 to 100 nm, more preferably in the range of 5 to 50 nm and a secondary particle size between 0.05 and 50 µm, more preferably between 0.1 and 1 µm. The particle size of the micronized double coated titanium dioxide particles having an inner inorganic silica coating and an outer silicone coating in the organic titanium dioxide dispersion is preferably in the range of 300 to 400 nm
The organic titanium dioxide dispersion may be prepared by known methods in the art e.g. by mixing C₁₂₋₁₅ alkyl benzoate with double coated micronized titanium dioxide particles in the presence of polyglyceryl-2 dipolyhydroxystearate. Preferably, a mixture of the C₁₂₋₁₅ alkyl benzoate and the polyglyceryl-2 dipolyhydroxystearate is prepared before the micronized double coated titanium dioxide particles are added. The preparation of the organic titanium dioxide dispersion can e.g. be performed with a roller milling, a ball milling, a homogenizer or sonic mixing.

The topical compositions according to the present invention can be prepared according to the state in the art. Preferred are topical compositions for the protection of the skin against UV-radiation such as topical sunscreen compositions.
The organic titanium dioxide dispersion is preferably incorporated into the topical compositions according to the invention in a concentration of 0.5 to 50% by weight, preferably 1 to 25 wt.-% (based on the dry powder).
The organic titanium dioxide dispersion can be incorporated directly into the topical compositions according to the invention without further preparatory measures according to known methods to a person skilled in the art.
The invention also relates to a method of preparation of a topical composition according to the invention characterized in that a dispersion consisting of micronized double coated titanium dioxide particles having an inner inorganic silica coating and an outer silicone coating, C₁₂₋₁₅ alkyl benzoate and polyglyceryl-2 dipolyhydroxystearate with all the definitions and preferences as outlined above is incorporated into a cosmetically acceptable carrier. The dispersion can either be added to the oil phase or at the end to the final formulation by known methods to a person skilled in the art.
The novel topical compositions exhibit clearly improved water resistance and thus improved protection against the harmful effects of solar rays even after bathing or sweating. Furthermore, the topical compositions show improved sun protection factors compared to the incorporation of double coated titanium dioxide particles as such (i.e. non dispersed).
Thus, the invention also relates to a method of improvement of the water resistance of micronized double coated titanium dioxide particles having an inner inorganic silica coating and an outer silicone coating in a topical composition characterized in that said particles are incorporated into the topical composition the form of a dispersion of said particles in C₁₂₋₁₅ alkyl benzoate and polyglyceryl-2 dipolyhydroxystearate.
The term "topical composition" as used herein refers in particular to cosmetic compositions that can be topically applied to mammalian keratinous tissue such as e.g. human skin or hair, particularly human skin.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic preparations as disclosed in A. Domsch, "Cosmetic Preparations", Verlag fur chemische Industrie (ed. H. Ziolkowsky), 4th edition, 1992.

The term cosmetically acceptable carrier refers to all carriers and/or excipients and/ or diluents conventionally used in topical compositions or preparations.

Preferably, the topical compositions according to the present invention are in the form of a suspension or dispersion in solvents or fatty substances, or alternatively in the form of an emulsion or micro emulsion (in particular of O/W- or W/O-type), PIT-emulsion, multiple emulsion (e. g. O/W/O- or W/O/W-type), pickering emulsion, hydrogel, alcoholic gel, lipogel, one- or multiphase solution or vesicular dispersion or other usual forms, which can also be applied by pens, as masks or as sprays. If the topical preparation is or comprises an emulsion it can also contain one or more anionic, nonionic, cationic or amphoteric surfactant(s).

In a particular embodiment, the topical compositions according to the invention are O/W emulsions. Preferably, the surfactant of the O/W emulsion are selected from the group of glycerylstearate in combination with ceteareth-20 and/ or ceteareth-25, ceteareth-6 in combination with stearylalcohol, cetylstearylalcohol in combination with PEG-40-ricinusoil and sodiumcetylstearylsulfate, triceteareth-4 phosphate, glycerylstearate, sodiumcetylstearylsulfate, lecithin trilaureth-4 phosphate, laureth-4 phosphate, stearic acid, propylenglycolstearate SE, PEG-25-hydrated ricinus oil, PEG-54-hydrated ricinus oil and/ or PEG-6 caprylic acid/caprinic acid glycerides, glyceryloleate in combination with propylenglycole, PEG-9-Stearate, PEG-20 Stearate, PEG-30-Stearate, PEG-40-stearate, PEG-100-stearate, ceteth-2, ceteth-20, polysorbate-20, polysorbate-60, polysorbate-65 and/ or polysorbate-100, glycerylstearate in combination with PEG-100 stearate, glycerylmyristate, glyceryllaurate, PEG-40-Sorbitanperoleat, Laureth-4, Ceteareth-3 and/ isostearylglycerylether, cetylstearylalkohol in combination with sodium cetylstearylsulfat, laureth-23 and/ or steareth-2, glycerylstearat in combination with PEG-30 stearate, PEG-40-stearate, glycol distearate, PEG-22-dodecyl glycol Copolymer, polyglyceryl-2-PEG-4-stearat, ceteareth-12, ceteareth-20, ceteareth-30, methyl-glucosesesquistearate, steareth-10 and/ or PEG-20-stearat, steareth-2 in combination with PEG-8 distearate, steareth-21, steareth-20, isosteareth-20, PEG-45/ dodecylglycol-copolymer, methoxy-PEG-22/dodecylglycol-copolymer, PEG-40-sorbitanperoleat, PEG-40-sorbitanperisostearats, PEG-20-glycerylstearats, PEG-20-glycerylstearats, PEG-8-bee wax, polyglyceryl-2-laurate, isostearyldiglycerylsuccinat, stearamidopropyl-PG-dimoniumchloridphosphat, ceteth-20, glycerylstearate SE, triethylcitrate, PEG-20-methylglucosesesquistearat, cetylphosphate, glycerylstearatcitrate, cetearyl sulfate, sorbitansesquioleate, triceteareth-4-phosphats, trilaureth-4-phosphate, polyglycerylmethylglucosedistearate, potassium cetylphosphate, polyglyceryl-3 methylglucose distearate, isosteareth-10, polyglyceryl-2-sesquiisostearate, ceteth-10, oleth-20 and/ or isoceteth-20, glycerylstearate in combination with ceteareth-20, ceteareth-12, cetylstearylalcohol and/ or cetylpalmitate, cetylstearylalcohol in combination with PEG-20 stearate, PEG-30-stearate, PEG-40-stearate and/ or PEG-100-stearate. Particularly preferred O/W surfactants are phosphate ester surfactants such as cetyl phosphate, potassium cetyl phosphate and/ or DEA cetyl phosphate, glycerylstearate, PEG-40 stearate, PEG-100 stearate in combination with glyceryl stearate, triglycerinmethylglucosedistearate, polyglyceryl-3 methylglucose distearate, cetearylglucoside, polyethylenglycol(21)stearylether, polyethylenglycol(2)stearylether, glycerylstearatcitrate, sodium cetearyl-sulfat, setearylalkohol, stearic acid and/ or sorbitanstearate.
Particularly preferred O/W surfactants according to the invention are phosphate ester surfactant, in particular selected from cetyl phosphate, potassium cetyl phosphate and/ or DEA cetyl phosphate and/ or PEG-100 stearate in combination with glyceryl stearate. The most preferred O/W surfactants according to the invention are the phosphate ester surfactants cetyl phosphate, potassium cetyl phosphate and/ or DEA cetyl phosphate
The oil phase of the O/W emulsions according to the invention preferably comprises oils selected from butylenglykoldicaprylat/-dicaprat, dicaprylylether, C₁₈₋₃₈-fatty acid triglyceride, dibutyladipate, cyclomethicone, 2-phenylethylbenzoat, isopropyl lauroyl sarcosinate as well as mixtures thereof. The amount of the oil (one or several) in the O/W emulsion according to the invention is generally selected in the range of 0.1 bis 40 wt.-%, in particular in the range of 1.0 to 30 wt.-%, most in particular in the range of 5% to 20 wt.-% with respect to the total weight of the topical composition.
In another particular embodiment, the topical compositions according to the invention are gels, as the incorporation of the titanium dioxide dispersion with the preferences and definitions as outlined above, in contrast to other titanium dioxide grades and titanium dioxide dispersions, surprisingly leads to an aesthetically pleasing appearance. Particular preferred are Pemulen Gels such as Pemulen TR Gels.

Preferred topical compositions according to the invention are sun care preparations such as in particular very water resistant sunscreens (also called waterproof sunscreens).

Topical compositions in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foam, a spray, a stick.

The topical cosmetic compositions of the invention can also contain usual cosmetic adjuvants and additives such as e.g. further UV-filter substances, preservatives, antioxidants, fatty substances, oils, water, alcohols, polyols, organic solvents, electrolytes, silicones, thickeners, film forming agents, softeners, emulsifiers, complexing agents, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, cosmetically active ingredients or any other ingredients, carriers and/or excipients or diluents conventionally formulated into cosmetic compositions. Such cosmetic ingredients commonly used in the skin care industry, which are suitable for use in the compositions of the present invention, are e.g. described in the CTFA Cosmetic Ingredient Handbook, Second Edition (1992) without being limited thereto.

The necessary amounts of the cosmetic and dermatological adjuvants and additives can - based on the desired product - easily be chosen by a skilled person in this field and will be illustrated in the examples, without being limited hereto.

Preferably, the topical compositions according to the invention comprise further UV filter substances which are preferably selected from conventional UVA and/or UVB and/ or broad spectrum UV-filter substances known to be added into topical compositions such as cosmetic or dermatological sun care products. Such UV-filter substances comprise all groups which absorb light in the range of wavelengths 400 nm to 320 nm (UVA) and 320 nm to 280 nm (UVB) or of even shorter wavelengths (UVC) and which are or can be used as cosmetically acceptable UV-filter substances. Such UV-filter substances are e.g. listed in the CTFA Cosmetic ingredient Handbook or "The Encyclopedia of violet Filters" (ISBN: 978-1-932633-25-2) by Nadim A. Shaath.
Suitable UV-filter substances may be organic or inorganic compounds. Exemplary organic UV-filter substances encompass e.g. acrylates such as e.g. 2-ethylhexyl 2-cyano-3,3-diphenylacrylate (octocrylene, PARSOL® 340), ethyl 2-cyano-3,3-diphenylacrylate; Camphor derivatives such as e.g. 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, terephthalylidene dicamphor sulfonic acid (Mexoryl® SX); Cinnamate derivatives such as e.g. ethylhexyl methoxycinnamate (PARSOL® MCX), ethoxyethyl methoxycinnamate, isoamyl methoxycinnamate as well as cinnamic acid derivatives bond to siloxanes; p-Aminobenzoic acid derivatives such as e.g. p-aminobenzoic acid, 2-ethylhexyl p-dimethylaminobenzoate, N-oxypropylenated ethyl p-aminobenzoate, glyceryl p-aminobenzoate; Benzophenones such as e.g. benzophenone-3, benzophenone-4, 2,2',4,4'-tetrahydroxy-benzophenone, 2,2'-dihydroxy-4,4'-dimethoxybenzophenone; Esters of benzalmalonic acid such as e.g. di-(2-ethylhexyl) 4-methoxybenzalmalonate; Organosiloxane compounds carrying chromophore groups such as e.g. polysilicones-15 (PARSOL® SLX), drometrizole trisiloxane (Mexoryl® XL); Salicylate derivatives such as e.g. isopropylbenzyl salicylate, benzyl salicylate, butyl salicylate, ethylhexyl salicylate (PARSOL® EHS, Neo Heliopan® OS), isooctyl salicylate or homomenthyl salicylate (homosalate, PARSOL® HMS, Neo Heliopan® HMS); Triazine derivatives such as e.g. ethylhexyl triazone (Uvinul® T-150), diethylhexyl butamido triazone (Uvasorb® HEB), bis-ethylhexyloxyphenol methoxyphenyl triazine (Tinosorb® S), 2,4,6-Tribiphenyl-4-yl-1,3,5 Triazine; Benzotriazole derivatives such as e.g. 2,2'-methylene-bis-(6-(2H-benzotriazole-2-yl)-4-(1,1,3,3,-tetramethylbutyl)-phenol (Tinosorb® M); Encapsulated UV-filters such as e.g. encapsulated ethylhexyl methoxycinnamate (Eusolex® UV-pearls) or microcapsules loaded with UV-filters as e.g. disclosed in EP 1471995; Dibenzoylmethane derivatives such as e.g. 4-tert.-butyl-4'-methoxydibenzoyl-methane (PARSOL® 1789), dimethoxydibenzoylmethane, isopropyldibenzoylmethane; Phenylene-1,4-bis-benzimidazolsulfonic acids or salts such as e.g. 2,2-(1,4-phenylene)bis-(1H-benzimidazol-4,6-disulfonic acid) (Neoheliopan AP); Amino substituted hydroxybenzophenones such as e.g. 2-(4-diethylamino-2-hydroxy-benzoyl)-benzoic acid hexylester (Aminobenzophenon, Uvinul® A Plus), 1,1'-(1,4-piperazinediyl)bis[1-[2-[4-(diethylamino)-2-hydroxybenzoyl]phenyl]-methanone; Benzoxazol-derivatives such as e.g. 2,4-bis-[5-1(dimethylpropyl)benzoxazol-2-yl-(4-phenyl)-imino]-6-(2-ethylhexyl)-imino-1,3,5-triazine [Uvasorb® K2A), Ethylhexyl Bis-Isopentylbenzoxazolyl phenyl Melamine and merocyanines as e.g. disclosed in DE10 2007 024 345 on page 4, paragraph 19 which are incorporated by reference herein
Further inorganic UV-filter substances may be present and encompass pigments such as e.g. microparticulated (micronized) Zink oxide. The term "microparticulated" refers to a particle size from 5 nm to 200 nm, particularly from 15 nm to 100 nm. The particles may also be coated by other metal oxides such as e.g. aluminum or zirconium oxides or by organic coatings such as e.g. polyols, methicone, aluminum stearate, alkyl silane. Such coatings are well known in the art.
In order to enhance the photostability of sun care products it may be desirable to add a photostabilizer. Exemplary photostabilizers known to a skilled person in the art encompass e.g. 3,3-diphenylacrylate derivatives such as e.g. octocrylene (PARSOL® 340) or Polyester-8 (Polycrylene®); Benzylidene camphor derivatives such as e.g. 4-methyl benzylidene camphor (PARSOL® 5000); Benzalmalonate derivatives such as e.g. polysilicones-15 (PARSOL® SLX) or diethylhexyl syringylidene malonate (Oxynex ST liquid); Dialkyl naphthalates such as diethylhexyl naphthalate (Corapan TQ) without being limited thereto. An overview on further stabilizers is e.g. given in 'SPF Boosters & Photostability of violet Filters', HAPPI, October 2007,p. 77-83 which is included herein by reference. The photostabilizers are generally used in an amount of 0.05 to 10 wt.-% with respect to the total weigh of the topical composition.
The additional UV-filter substances are generally present in the compositions according to the invention in proportions ranging from 0.1 to 30 wt.-%, preferably ranging from 0.2 to 15 wt.-%, most preferably ranging from 0.5 to 10 wt.-% with respect to the total weigh of the composition.
Generally, the amount of each UV-filter substance in the topical compositions according to the invention is selected in the range of 0.1 to 10 wt.-%, preferably in the range of 0.2 to 7 wt.-%, most preferably in the range of 0.5 to 5 wt.-% with respect to the total weigh of the topical composition.

The total amount of UVA-filter substance(s), in particular of butyl methoxydibenzoylmethane, in the topical compositions according to the invention is preferable selected in the range of 0.5 to 7 wt.-%, in particular in the range of 1 to 6 wt.-%, most particular in the range of 2 to 5 wt.-% with respect to the total weight of the topical composition.
The total amount of UV-filter substances in the topical compositions according to the invention is preferably in the range of 1 to 40 wt.-%, preferably in the range of 5 to 30 wt.-%, in particular in the range of 20 to 30 wt.-% with respect to the total weight of the topical composition.
Preferred further UVB-filter substances to be used in the topical compositions according to the invention encompass polysilicones-15, octocrylene, ethylhexyl methoxycinnamate, ethyl hexylsalicylate and/ or homosalate.
Preferred broadband UV-filter substances to be used in the topical compositions according to the invention encompass unsymmetrical s-triazine derivatives such 2,4-Bis-{[4-(2-ethyl-hexyloxy)-2-hydroxy]-phenyl}-6-(4-methoxyphenyl)-1,3,5-triazin and 2,4,6-Tris-(biphenyl) 1,3,5-triazine, certain benzophenones such as e.g. 2-Hydroxy-4-methoxy-benzophenon and/ or 2,2'-Methylen-bis-(6-(2H-benzotriazol-2-yl)-4-(1,1,3,3-tetramethyl-butyl)-phenol).
The preferred UVA-filter substance to be used in the topical compositions according to the invention is butyl methoxydibenzoylmethane (commercially available as PASRSOL® 1789 by DSM Nutritional Products Ltd.).
Preferred topical compositions according to the invention further comprise one or several antioxidant(s). Suitable antioxidants for the incorporation into the topical compositions according to the invention are all antioxidant suitable for cosmetic applications. Particular preferred are water soluble antioxidants such as vitamins such as e.g. ascorbic acid as well as derivatives thereof such as e.g. ascorbyl phosphate such as Stay C (sodium ascorbyl monophosphate) from DSM Nutritional Products Ltd.
Further preferred antioxidants are BHT, vitamin E and derivatives thereof as well as vitamin A and derivatives thereof.

The amount of antioxidant (one or several) in the topical compositions according to the invention is preferably selected in the range of 0.001 to 30 wt.-%, in particular in the range of 0.05 to 20 wt.-%, most particular in the range of 0.1 to 10 wt.-%, with respect to the total amount of the topical composition.
If a vitamin E derivative is used in the topical compositions according to the invention preferably tocopheryl acetate is used. Tocopheryl acetate may be present in the topical preparations in an amount from 0.05 to 25 wt.-%, in particular 0.5 to 5 wt.-%. Another vitamin E derivative of interest is tocopheryl linoleate. Tocopheryl linoleate may be present in the skin care composition in an amount from 0.05 to 25 wt.-% in particular 0.5 to 5 wt.-%.
Vitamin A and/or its derivatives in particular retinoid derivatives such as retinyl palmitate or retinyl propionate is preferably used in the topical preparations according to the invention in an amount of 0.01 to 5 wt.-%, in particular 0.01 to 0.3 wt.-%.
Preferably, the topical compositions according to the invention further comprise a fatty alcohol, such as in particular cetyl alcohol, cetearyl alcohol and/ or behenyl alcohol. The total amount of one or several fatty alcohols on the topical compositions according to the invention is preferably selected in the range of 0.1 to 10.0 wt.-%, in particular in the range of 0.5 to 6.0 wt.-% with respect to the total weight of the topical composition. Preferred examples of cosmetically active ingredients for the incorporation into topical compositions according to the invention encompass vitamin B₆, vitamin B₁₂, biotin, co-enzyme Q10, EGCG, alpha-liponic acid, phytoen, hydroxytyrosol and/or olive extract, shea butter, algae extract, cocoa butter, aloe extract, jojoba oil, echinacea extract, chamomile extract, alpha-glucosylrutin, carnitin, carnosine, natural and/ or synthetic isoflavanoids, creatin, taurin, alanine, glycyrrhetinic acid, glycyryca glabra and/ or glycyrrhiza inflata.
The cosmetically active ingredient is typically included in an amount of at least 0.001 wt. % based on the total weight of the topical preparation. Generally, an amount of 0.001 wt. % to 30 wt. %, preferably from 0.001 wt. % to 10 wt. % of an additional cosmetically active agent is used. Further preferred the topical compositions according to the invention further comprise a moisturizer. Moisturizers are chemical agents specially designed to make the external layers of the skin (epidermis) softer and more pliable by increasing its hydration (water content) which can be determined e.g. by measuring the transepidermal water loss (TEWL). Suitable moisturizer according to the invention are for example glycerin, lactic acid and/ or lactate such as in particular sodium lactate, butyleneglycol, propyleneglycol, biosaccaride gum-1, glycine soja, ethylhexyloxyglycerine, pyrrolidoncarbonic acid and/or urea.

Suitable preservatives according to the invention encompass for example formaldehyde releasing chemicals such as e.g. DMDM Hydantoin (e.g. as Glydant by Lonza), iodopropylbutylcarbamate (e.g. as Glycacil-L, Glycacil-S by Lonza and/ or Dekaben LMB by Jan Dekker), parabens (e.g. p-hydroxybenzoic acid alkyl ester such as methyl-, ethyl-, propyl- and/ or butylparaben), phenoxyethanol, ethanol, benzoic acid without being limited thereto.

Preferably, the topical compositions according to the invention comprise a thickener in particular if the topical composition is in the form of an emulsion to assist in making the consistency of a product suitable. Preferred thickeners are aluminiumsilicates, xanthan gum, hydroxypropylmethylcellulose, polyacrylates such as carbopole® (e.g. Carbopole 980, 981, 1382, 2984, 5984) or mixtures thereof. Further preferred thickeners encompass acrylate/C₁₀₋₃₀ alkyl acrylate copolymers (such as e.g. Pemulen TR 1, Pemulen TR 2, Carbopol 1328 by. NOVEON) as well as Aristoflex AVC (INCI: Ammonium Acryloyldimethyltaurate/VP Copolymer).

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

The cosmetic and/ or dermatological compositions according to the invention have a pH in the range of 3-10, preferably in the range of pH of 4-8, most preferred in the range of pH 4-7.
The invention further refers to the use of a topical composition according to the invention for the protection of human skin and/ or hair against UV-radiation

The following examples are provided to further illustrate the processes and compositions of the present invention. These examples are illustrative only and are not intended to limit the scope of the invention in any way.

### Example 1: Water resistance test

Four sun care emulsions comprising either Parsol® TX or a dispersion consisting of 50 wt.-% of Parsol® TX, C₁₂-C₁₅ Alkylbenzoate (43 wt.-%) and PGPH (7wt.-%) at two different concentration levels have been prepared according to standard methods.
For each formulation four PMMA-plates have been prepared.

### In vitro SPF Measurement:

The in vitro SPF's were measured using a MUT Helias" (MUT Aviation-Technology GmbH) on PMMA plates (2.5 x 50 x 50 mm) at four different points

### Application:

18.7 mg ± 0.2 mg of the respective formulation was applied with a syringe dot wise on the PMMA-plate and spread evenly on the plate by rubbing with a finger which was saturated beforehand with the respective formulation. The sample was allowed to settle for 30 min in an oven at 43°C and the in vitro SPF (initial) was measured.

Water immersion: The measurements have been performed using 3l beakers filled with 2.5 l of room tempered osmosed water. After having fixed the PMMA plates with a spacer to the wall of the beakers the water was stirred constantly using a magnetic stirrer (450 1/min) in order to assure an optimum lamellar flow of the water around the plates. After 20 min immersion, the samples were taken out and the excessive water was removed at 43°C for 30 min. After reaching room temperature, the in vitro SPF was measured again. The results are summarized in Table 1.

**Table 1**

| **Ingredients** | **Reference** | **Dispersion** | **Reference** | **Dispersion** |
|---|---|---|---|---|
| Estol 3650 | 2.0 | 2.0 | 2.0 | 2.0 |
| Lanette O | 1.0 | 1.0 | 1.0 | 1.0 |
| Myritol 318 | 15.0 | 15.0 | 15.0 | 15.0 |
| Finsolv TN | 10.0 | 5.0 | 10.0 | 0.0 |
| Dow Corning Cosmetic | 2.0 | 2.0 | 2.0 | 2.0 |
| Amphsiol K | 2.0 | 2.0 | 2.0 | 2.0 |
| Phenonip | 0.8 | 0.8 | 0.8 | 0.8 |
| BHT | 0.05 | 0.05 | 0.05 | 0.05 |
| PARSOL TX Dispersion | - | 10.0 | - | 20.0 |
| PARSOL TX | 5.0 | - | 10.0 | - |
| Pemulen TR 1 | 0.3 | 0.3 | 0.3 | 0.3 |
| Glycerin | 8.0 | 8.0 | 8.0 | 8.0 |
| EDTA | 0.1 | 0.1 | 0.1 | 0.1 |
| TEA | 0.4 | 0.4 | 0.4 | 0.4 |
| water | 46.7 | 46.7 | 51.7 | 51.7 |
| SPF before water immersion | 10.0 ± 1.6 | 11.1±1.7 | 19.8 ± 4.0 | 24.4 ± 9 |
| SPF after water immersion | 5.2 ± 0.2 | 7.5 ± 0.8 | 15.1 ± 3.1 | 21.3 ± 4.8 |
| Δ SPF (before - after) | 4.8 | 3.6 | 4.7 | 3.1 |
| % decrease of SPF | -47.00% | -32.50% | -23.73% | -12.70% |

As can be retrieved from table 1 next to an increase of the SPF the water resistance is significantly improved by the use of the titanium dioxide dispersion illustrated by a significantly reduced decrease of the in vitro SPF after the wash-off procedure.

### Example 2 Sunscreen preparations

**O/W Sunscreen**

| **Phase** | ***Ingredients*** | ***INCI Name*** | ***Wt.-%*** |
|---|---|---|---|
| A | Dermofeel BGC | Butylene Glycol Dicaprylate/Dicaprate | 3.00 |
| | PARSOL® 1789 | Butyl Methoxydibenzoylmethane (Avobenzone; USAN) | 4.50 |
| | PARSOL® 340 | Octocrylene (Octocrilene; USAN) | 4.00 |
| | Eutanol G | Octyldodecanol | 3.00 |
| | Cetiol OE | Dicaprylyl Ether | 3.00 |
| | Tinosorb S | Bis-Ethylhexyloxyphenol Methoxyphenyl Triazine | 2.00 |
| | Cetiol CC | Dicaprylyl Carbonate | 2.00 |
| | Imwitor 372 P Schuppen | Glyceryl Stearate Citrate | 1.00 |
| | Lanette 18 | Stearyl Alcohol | 1.00 |
| | Lipocire Na 10 Pastilles | Hydrogenated Coco-Glycerides | 1.00 |
| | dl-alpha -Tocopheryl Acetate | Tocopheryl Acetate | 0.50 |
| | Antaron V-216 | VP/Hexadecene Copolymer | 1.00 |
| | BHT | Butylated Hydroxytoluene | 0.05 |
| | Phenonip | Phenoxyethanol & Methylparaben & Ethylparaben & Butylparaben & Propylparaben & Isobutylparaben | 0.80 |
| | PARSOL TX50 AB | C12-15 Alkyl Benzoate&%Titanium Dioxide&Dimethicon&Silica&Polyglyceryl-2 Dipolyhydroxystearate | 10.00 |
| B | Glycerin | Glycerin | 8.00 |
| | Keltrol CG-T | Xanthan Gum | 0.30 |
| | Pemulen TR-1 | Acrylates/C10-30 Alkyl Acrylate Crosspolymer | 0.30 |
| | Edeta BD | Disodium EDTA | 0.10 |
| | Water dem. | Aqua | Ad 100 |
| C | Sensiva SC 50 | Ethylhexylglycerin | 0.50 |
| | Ethanol | Alcohol | 4.00 |
| | Triethanolamine (T.E.A.) | Triethanolamine | 0.35 |

**Pemulen Gel**

| ***Phase*** | ***Ingredients*** | ***INCI Name*** | ***Wt***.**-*%*** |
|---|---|---|---|
| A | Myritol 318 | Caprylic/Capric Triglyceride | 5.0 |
| | Phenonip | Phenoxyethanol & Methylparaben & Ethylparaben & Butylparaben & Propylparaben & Isobutylparaben | 0.8 |
| | BHT | Butylated Hydroxytoluene | 0.1 |
| | PARSOL TX50 AB | C12-15 Alkyl Benzoate&%Titanium Dioxide&Dimethicon&Silica&Polyglyceryl-2 Dipolyhydroxystearate | 10 |
| | Pemulen TR 1 | Acrylates/C10-30 Alkyl Acrylate Crosspolymer | 0.6 |
| | EDTA | Disodium EDTA | 0.1 |
| | KOH 30% | Potassium Hydroxide | 1.70 |
| | water | water | Ad 100 |

## Claims

1. A topical composition comprising in a cosmetically acceptable carrier an organic titanium dioxide dispersion, **characterized in that** the dispersion consists of micronized double coated titanium dioxide particles having an inner inorganic silica coating and an outer silicone coating, C₁₂₋₁₅ alkyl benzoate and polyglyceryl-2 dipolyhydroxystearate.

2. The topical composition according to claim 1, **characterized in that** the dispersion consists of 10-80 wt.-% of double coated titanium dioxide particles, 10-80 wt.-% of C₁₂₋₁₅ alkyl benzoate and 0.5-20 wt.-% of polyglyceryl-2 dipolyhydroxystearate.

3. The topical composition according to claim 1, **characterized in that** the dispersion consists of 25-60 wt.-% of double coated titanium dioxide particles 30-70 wt.-% of C₁₂₋₁₅ alkyl benzoate and 2-15 wt.-% of polyglyceryl-2 dipolyhydroxystearate.

4. The topical composition according to any one of the claims 1 to 3, **characterized in that** the outer coating of the titanium dioxide particles is polydimethylsiloxane.

5. The topical composition according to any one of the claims 1 to 4, **characterized in that** the double coated titanium dioxide particles are in the form of a white powder, consisting of titanium oxide of rutile crystal structure coated with silica and polydimethylsiloxane.

6. The topical composition according to any one of the claims 1 to 5, **characterized in that** the ratio (w/w) of the double coated titanium dioxide particles to polyglyceryl-2 dipolyhydroxystearate is selected in the range of 20 to 1 to 10 to 1.

7. The topical composition according to any one of claims 1 to 6, **characterized in that** the organic titanium dioxide dispersion (based on the dry powder) is present in an amount of 0.5 to 50 wt.-% based on the total weight of the topical composition.

8. The topical composition according to claim 7 **characterized in that** said composition further comprises from 0.5 - 7 wt.-% of a dibenzoylmethane derivative, which is butyl methoxydibenzoylmethane.

9. The topical composition according to any one of claims 1 to 8 **characterized in that** said composition is in the form of an O/W emulsion or a gel.

10. Topical composition according to any of claims 1 to 9 for use in the protection of human skin against UV-radiation.

11. Use of a topical composition according to any of claims 1 to 9 for the protection of hair against UV-radiation.

12. Method of improvement of the water resistance of micronized double coated titanium dioxide particles having an inner inorganic silica coating and an outer silicone coating in a topical composition according to any one of claims 1 to 9 **characterized in that** said particles are incorporated into the topical composition in the form of a dispersion of said particles in C₁₂₋₁₅ alkyl benzoate and polyglyceryl-2 dipolyhydroxystearate.

13. Method of preparation of a topical composition according to any one of claims 1 to 9 **characterized in that** a dispersion consisting of micronized double coated titanium dioxide particles having an inner inorganic silica coating and an outer silicone coating, C₁₂₋₁₅ alkyl benzoate and polyglyceryl-2 dipolyhydroxystearate is incorporated into a cosmetically acceptable carrier.

## Patentansprüche

1. Topische Zusammensetzung, umfassend eine organische Titandioxiddispersion in einem kosmetisch annehmbaren Träger, **dadurch gekennzeichnet, dass** die Dispersion aus mikronisierten, doppelt beschichteten Titandioxidpartikeln, die eine innere anorganische Siliciumdioxidbeschichtung und eine äußere Siliconbeschichtung aufweisen, C₁₂₋₁₅Alkylbenzoat und Polyglyceryl-2-dipolyhydroxystearat besteht.

2. Topische Zusammensetzung gemäß Anspruch 1, **dadurch gekennzeichnet, dass** die Dispersion aus 10-80 Gew.-% an doppelt beschichteten Titandioxidpartikeln, 10-80 Gew.-% C₁₂₋₁₅Alkylbenzoat und 0,5-20 Gew.-% Polyglyceryl-2-dipolyhydroxystearat besteht.

3. Topische Zusammensetzung gemäß Anspruch 1, **dadurch gekennzeichnet, dass** die Dispersion aus 25-60 Gew.-% an doppelt beschichteten Titandioxidpartikeln, 30-70 Gew.-% C₁₂₋₁₅Alkylbenzoat und 2-15 Gew.-% Polyglyceryl-2-dipolyhydroxystearat besteht.

4. Topische Zusammensetzung gemäß einem der Ansprüche 1 bis 3, **dadurch gekennzeichnet, dass** die äußere Beschichtung der Titandioxidpartikel Polydimethylsiloxan ist.

5. Topische Zusammensetzung gemäß einem der Ansprüche 1 bis 4, **dadurch gekennzeichnet, dass** die doppelt beschichteten Titandioxidpartikel in der Form eines weißen Pulvers vorliegen, das aus Titanoxid mit Rutil-Kristallstruktur, das mit Siliciumdioxid und Polydimethylsiloxan beschichtet ist, besteht.

6. Topische Zusammensetzung gemäß einem der Ansprüche 1 bis 5, **dadurch gekennzeichnet, dass** das Verhältnis (w/w) der doppelt beschichteten Titandioxidpartikel zu Polyglyceryl-2-dipolyhydroxystearat in dem Bereich von 20 zu 1 bis 10 zu 1 ausgewählt ist.

7. Topische Zusammensetzung gemäß einem der Ansprüche 1 bis 6, **dadurch gekennzeichnet, dass** die organische Titandioxiddispersion (bezogen auf das trockene Pulver) in einer Menge von 0,5 bis 50 Gew.-% bezogen auf das Gesamtgewicht der topischen Zusammensetzung vorhanden ist.

8. Topische Zusammensetzung Anspruch 7, **dadurch gekennzeichnet, dass** die Zusammensetzung ferner von 0,5 bis 7 Gew.-% an einem Dibenzoylmethanderivat, das Butylmethoxydibenzoylmethan ist, umfasst.

9. Topische Zusammensetzung gemäß einem der Ansprüche 1 bis 8, **dadurch gekennzeichnet, dass** die Zusammensetzung in der Form einer O/W-Emulsion oder eines Gels vorliegt.

10. Topische Zusammensetzung gemäß einem der Ansprüche 1 bis 9 für die Verwendung zum Schützen menschlicher Haut gegen UV-Strahlung.

11. Verwendung einer topischen Zusammensetzung gemäß einem der Ansprüche 1 bis 9 zum Schützen von Haar gegen UV-Strahlung.

12. Verfahren zum Verbessern der Wasserbeständigkeit von mikronisierten, doppelt beschichteten Titandioxidpartikeln, die eine innere anorganische Siliciumdioxidbeschichtung und eine äußere Siliconbeschichtung aufweisen, in einer topischen Zusammensetzung gemäß einem der Ansprüche 1 bis 9, **dadurch gekennzeichnet, dass** die Partikel in der Form einer Dispersion der Partikel in C₁₂₋₁₅Alkylbenzoat und Polyglyceryl-2-dipolyhydroxystearat in die topische Zusammensetzung einverleibt werden.

13. Verfahren zum Herstellen einer topischen Zusammensetzung gemäß einem der Ansprüche 1 bis 9, **dadurch gekennzeichnet, dass** eine Dispersion, die aus mikronisierten, doppelt beschichteten Titandioxidpartikeln, die eine innere anorganische Siliciumdioxidbeschichtung und eine äußere Siliconbeschichtung aufweisen, C₁₂₋₁₅Alkylbenzoat und Polyglyceryl-2-dipolyhydroxystearat besteht, in einen kosmetisch annehmbaren Träger einverleibt wird.

## Revendications

1. Composition topique comprenant une dispersion organique de dioxyde de titane dans un véhicule cosmétiquement acceptable, **caractérisée en ce que** la dispersion se compose de particules de dioxyde de titane micronisées et doublement enrobées ayant un revêtement de silice inorganique interne et un revêtement de silicone externe, d'un benzoate d'alkyle en C₁₂ à C₁₅ et de 2-dipolyhydroxystéarate de polyglycéryle.

2. Composition topique selon la revendication 1, **caractérisée en ce que** la dispersion se compose de 10 à 80 % en poids de particules de dioxyde de titane doublement enrobées, de 10 à 80 % en poids de benzoate d'alkyle en C₁₂ à C₁₅ et de 0,5 à 20 % en poids de 2-dipolyhydroxystéarate de polyglycéryle.

3. Composition topique selon la revendication 1, **caractérisée en ce que** la dispersion se compose de 25 à 60 % en poids de particules de dioxyde de titane doublement enrobées, de 30 à 70 % en poids de benzoate d'alkyle en C₁₂ à C₁₅ et de 2 à 15 % en poids de 2-dipolyhydroxystéarate de polyglycéryle.

4. Composition topique selon l'une quelconque des revendications 1 à 3, **caractérisée en ce que** le revêtement externe des particules de dioxyde de titane est du polydiméthylsiloxane.

5. Composition topique selon l'une quelconque des revendications 1 à 4, **caractérisée en ce que** les particules de dioxyde de titane doublement enrobées sont sous la forme d'une poudre blanche, se composant d'oxyde de titane ayant la structure cristalline du rutile enrobé de silice et de polydiméthylsiloxane.

6. Composition topique selon l'une quelconque des revendications 1 à 5, **caractérisée en ce que** le rapport (m/m) des particules de dioxyde de titane doublement enrobées au 2-dipolyhydroxystéarate de polyglycéryle est choisi dans la plage allant de 20/1 à 10/1.

7. Composition topique selon l'une quelconque des revendications 1 à 6, **caractérisée en ce que** la dispersion organique de dioxyde de titane (par rapport à la poudre sèche) est présente en une quantité de 0,5 à 50 % en poids par rapport au poids total de la composition topique.

8. Composition topique selon la revendication 7, **caractérisée en ce que** ladite composition comprend en outre de 0,5 à 7 % en poids d'un dérivé de dibenzoylméthane, qui est le butylméthoxydibenzoylméthane.

9. Composition topique selon l'une quelconque des revendications 1 à 8, **caractérisée en ce que** ladite composition est sous la forme d'une émulsion H/E ou d'un gel.

10. Composition topique selon l'une quelconque des revendications 1 à 9, destinée à être utilisée dans la protection de la peau humaine contre un rayonnement UV.

11. Utilisation d'une composition topique selon l'une quelconque des revendications 1 à 9 pour la protection des cheveux contre un rayonnement UV.

12. Procédé d'amélioration de la résistance à l'eau de particules de dioxyde de titane micronisées et doublement enrobées ayant un revêtement de silice inorganique interne et un revêtement de silicone externe dans une composition topique selon l'une quelconque des revendications 1 à 9, **caractérisé en ce que** lesdites particules sont incorporées dans la composition topique sous la forme d'une dispersion desdites particules dans un benzoate d'alkyle en C₁₂ à C₁₅ et du 2-dipolyhydroxystéarate de polyglycéryle.

13. Procédé de préparation d'une composition topique selon l'une quelconque des revendications 1 à 9, **caractérisé en ce qu'**une dispersion se composant de particules de dioxyde de titane micronisées et doublement enrobées ayant un revêtement de silice inorganique interne et un revêtement de silicone externe, d'un benzoate d'alkyle en C₁₂ à C₁₅ et de 2-dipolyhydroxystéarate de polyglycéryle est incorporée dans un véhicule cosmétiquement acceptable.
